# EUROPEAN PATENT APPLICATION

(11) **EP 3 901 165 A1**
(43) Date of publication of application: **27.10.2021**
(21) Application number: 19886339.1
(22) Date of filing: 10.04.2019
(51) Int. Cl.: C07K 14/415, A61K 38/16, A61P 9/10, A61P 25/28, A61P 39/06

(54) **MULTI-DISULFIDE BRIDGE LONG-CHAIN PEPTIDE HAVING NEUROPROTECTIVE ACTIVITY, PHARMACEUTICAL COMPOSITION AND USES THEREOF**

(30) Priority: 25.11.2018 CN 201811411698
(71) Applicant: Dalian Institute Of Chemical Physics, Chinese Academy Of Sciences, Liaoning 116023 (CN); Nantong University, Nantong, Jiangsu 226019 (CN)
(72) Inventor: ZHANG, Xiaozhe, Dalian, Liaoning 116023 (CN); DING, Fei, Nantong, Jiangsu 226019 (CN); CHENG, Qiong, Nantong, Jiangsu 226019 (CN); GU, Xiaosong, Nantong, Jiangsu 226019 (CN); LIANG, Xinmiao, Dalian, Liaoning 116023 (CN); BAI, Yunpeng, Dalian, Liaoning 116023 (CN); YAO, Dengbing, Nantong, Jiangsu 226019 (CN); YUAN, Ying, Nantong, Jiangsu 226019 (CN); WANG, Caiping, Nantong, Jiangsu 226019 (CN); YANG, Jian, Nantong, Jiangsu 226019 (CN); YU, Shu, Nantong, Jiangsu 226019 (CN)
(74) Representative: Nederlandsch Octrooibureau
(86) International application number: PCT/CN2019/082056
(87) International publication number: WO 2020/103382

(57) **Abstract**

The present invention relates to a multi-disulfide-bond long-chain peptide with neuroprotective activity, a pharmaceutically acceptable salt thereof and a pharmaceutical composition having the multi-disulfide-bond long-chain peptide. The multi-disulfide-bond long-chain peptides can reduce cellular calcium influx by inhibiting glutamate receptors to protect cortical neurons from excitotoxicity induced by glutamate, and are a type of effective neuroprotective agents.

## Description

### Technical Field

The present invention relates to the field of biotechnology and pharmaceutical industry, and particularly relates to design and acquisition of a multi-disulfide-bond long-chain peptide with neuroprotective activity. The multi-disulfide-bond long-chain peptide is used for treatment for neuroprotective purpose.

### Background

Stroke has become one of the main causes that lead to death and disability, wherein 67.3-80.5% belongs to ischemic strokes, i.e., brain damage caused by decrease of cerebral blood flow due to thrombi or emboli that block the cerebral artery or its branches. At present, drugs for clinically treating the stroke, such as tissue-type plasminogen activator-alteplase, can realize revascularization and restore blood flow. The penumbra region cannot be converted into a central region, and it is possible to use neuroprotective agents to save the penumbra cells. However, so far, there is no effective neurocyte protective agent clinically and no therapeutic drug that can promote the recovery of nerve function. Despite decades of efforts, the understanding of endogenous neuroprotective molecules and mechanisms induced by the stroke is still very limited (Stroke epidemiology: A review of population-based studies of incidence, prevalence, and case-fatality in the late 20th century. Feigin VL, Lawes CM, Bennett DA, Anderson CS(2003), Lancet Neurol 2: 43-53; The science of stroke: mechanisms in search of treatments. Moskowitz MA,Lo EH, Iadecola C(2010).Neuron 67:181-198).

Researches have shown that glutamate-mediated excitatory toxicity is regarded as a major and key part in the cascade reaction of brain damage caused by cerebral ischemia/reperfusion. The mechanisms of glutamate release in ischemic brain injury are as follows: (1) extracellular glutamate accumulation triggered by glutamate transporters is caused due to the decrease in transporter protein expression, reverse reversal of glutamate transporter and the reduction of reuptake capability of the glutamate transporters. (2) Astrocytes can release glutamate through calcium-dependent exocytosis. Therefore, under ischemic conditions, this release mode may be disordered, causing increase of glutamate release and excessive activation of N-methyl-D-aspartic acid (NMDA) receptor, which is the main step of causing neuronal damage after ischemic stroke. (3) Cerebral ischemia leads to swelling of the astrocytes, further causes cerebral edema, and then causes the release of glutamate. (4) Activation of phospholipase A2 and phospholipase C and phospholipid hydrolysis lead to the imbalance and deterioration of the cytoplasmic membrane. The high concentration difference of amino acids on both sides of the membrane leads to transmembrane dispersion, causing the release of the amino acids. Therefore, there are at least four different mechanisms in the glutamate release mode caused by ischemia. In the early stage, due to Ca²⁺ influx and endoplasmic reticulum/mitochondrial Ca²⁺ outflux, intracellular Ca²⁺ increases, which induces calcium-dependent vesicular release. Then, glutamate transporter, swelling and phospholipase activation have important effects on glutamate release induced by ischemia (Inhibition of glial glutamate transporter glt-1 augments brain edema after transient focal cerebral ischemia in mice. Namura S, Maeno H, Takami S, Jiang XF, Kamichi S, Wada K, Nagata I (2002), Neurosci Lett 324: 117-120; Glutamate-mediated astrocyte-neuron signalling. Parpura V, Basarsky TA, Liu F, Jeftinija K, Jeftinija S, Haydon PG(1994), Nature 369: 744-747; Inhibition of ischemia-induced glutamate release in rat striatum by dihydrokinate and an anion channel blocker. Seki Y, Feustel PJ, Keller RW, Tranmer BI, Kimelberg HK(1999), Stroke 30: 433-440).

In the research of neuroprotective drugs, NMDA receptor blockers have always been the target of the research. The NMDA receptor is a heteromer composed of three different subunits of NR1, NR2 and NR3, wherein eight of NR1 subunits are derived from different splicing isomers of a single gene, while four different types of NR2 subunits (NR2A, NR2B, NR2C and NR2D) and two different types of NR3 subunits (NR3A and NR3B) are encoded by six different genes. A typical NMDA receptor comprises two NR1 subunits with a glycine binding site and two NR2 subunits with a glutamate binding site, wherein the latter can be replaced by the NR3 subunit. At the same time, NR1 is the basic subunit of the NMDA receptor, and the NR2 subunit makes the NMDA receptor form a diversified structure and exhibit different receptor functions. Recent researches have suggested that activation of the NMDA receptors containing different types of NR2 subunits has different effects on neuronal damage. The activation of the NMDA receptors containing NR2A subunits can promote the survival of neurons, and can protect NMDA receptor-mediated or non-NMDA receptor-mediated neuronal damage. On the contrary, the activation of the NMDA receptors containing NR2B subunits triggers excitotoxicity, leading to neuronal apoptosis. On the other hand, the location of the neuron where the activated NMDA receptor is located also affects its effect in the process of neuronal damage. The activation of the NMDA receptors at synapses has an anti-apoptosis effect, but the activation of the NMDA receptors at extrasynaptic sites can trigger cell death. This result is also confirmed by the result of whole gene expression map. This may explain the fact that the efficacy of non-selective NMDA receptor blockers in clinical application is not satisfactory, and also provides a scientific basis for searching and finding specific and effective NMDA receptor-based neuroprotective drugs (NMDA receptor subunits: Function and pharmacology. Paoletti P, Neyton J(2007) 7:39-47; The dichotomy of NMDA receptor signaling. Papadia S,Hardingham GE (2007), Neuroscientist 13:572-579; NMDA receptor subunits have differential roles in mediating excitotoxic neuronal death both in vitro and in vivo. Liu Y, Wong TP, Aarts M, Rooyakkers A, Liu L, Lai TW, Wu DC, Lu J, Tymianski M, Craig AM, Wang YT(2007), J Neurosci 27:2846-2857; Docoding NMDA receptor signaling: Identification of genomic programs specifying neuronal survival and death. Zhang SJ, Steijaert MN, Lau D, Schutz G, Delucinge-Vivier C(2007), Neuron 53:549-562.).

In recent years, the research of traditional Chinese medicine has been more favored by researchers. Achyranthes bidentata Blume is one of the frequently-used Chinese herbal medicines. It has the function of promoting blood circulation to remove blood stasis, and is also used for treatment of the stroke. The extract of Achyranthes bidentata Blume can promote peripheral nerve regeneration, has a protective effect on ischemic brain injury, can inhibit the apoptosis of PC12 cells due to serum deprivation, and is related to Bax and Caspase-3, without toxic and side effects. Researches have shown that the active component isolated from the extract of Achyranthes bidentata Blume is a polypeptide component, which is named A.bidentata polypeptide (referred to by ABPPk2). The first-order sequence of ABPPk2 has been identified by Edman degradation and mass spectrometry, and the pharmacological research of ABPPk2 has been conducted. At the same time, with the ABPPk2 as a basic sequence structure, a structural domain is determined through the interaction with NMDA2B receptors; based on the ABPPk2 structural domain, derivative modification is performed, so that new technologies of pharmacokinetics, biochores, stability and applications in preclinical scenes are improved (Stroke therapy in traditional Chinese medicine (TCM): Prospects for drug discovery and development. Gong X, Sucher NJ.(2002), Phytomedicine 9:478-484;The repair effects of achyranthes bidentata extract on the crushed common personal nerve of rabbits. Ding F, Cheng Q, Gu X(2007), Fitoterapia 79:161-167;Herbal medicine in stroke: Does it have a future? Feigin VL.(2007), Stroke 38:1734-1736.).

### Summary

The present invention provides a multi-disulfide-bond long-chain peptide with neuroprotective activity to solve the above problems. In the present invention, through the extraction and purification of the active peptides of ABPPk2, structural characterization, docking with the NMDA2B receptor molecules and derivative modification based on the structural domain, the multi-disulfide-bond long-chain peptide is used in related aspects of central nerve protective agents of ischemic stroke, neuroprotection caused by ischemia and hypoxia, and nerve growth promotion.

A neuroprotective agent of the multi-disulfide-bond long-chain peptide is provided. The primary sequence of amino acid of ABPPk2 is: Cys-Leu-Glu-Ser-Gly-Thr-Ser-Cys-Ile-Pro-Gly-Ala-Gln-His-Asn-Cys-Cys-Ser-Gly-Val-C ys-Val-Pro-Ile-Val-Thr-Ile-Phe-Tyr-Gly-Val-Cys-Tyr (i.e., CLESGTSCIPGAQHNCCSGVCVPIVTIFYGVCY).

In one embodiment, the ABPPk2 is obtained by extraction, enrichment and purification.

In one embodiment, the primary sequence of the amino acid of the ABPPk2 is characterized by Edman degradation and tandem mass spectrometry.

In one embodiment, the disulfide bond connection mode of the ABPPk2 is characterized by stepwise reduction and derivatization.

In one embodiment, NMR characterizes the three-dimensional spatial structure of the ABPPk2.

In one embodiment, the ABPPk2 is docked with the NMDA2B receptor molecule to determine the structural domain.

In one embodiment, the effect of the ABPPk2 on NMDA damage of primary cultured hippocampal neurons is provided.

In one embodiment, the protective effect of the ABPPk2 on hypoxic-ischemic damage of primary cultured SD fetal rat cortical neurons is provided.

In one embodiment, the effect of the ABPPk2 on the growth of primary cultured hippocampal neurons is provided.

In one embodiment, the multi-disulfide-bond long-chain peptide of claim 1 is simulated by homology modeling.

In one embodiment, the multi-disulfide-bond long-chain peptide of claim 1 is obtained by synthesis.

The peptide may adopt different structures. One or more of possible structures of the peptide may have specific biological importance. In order to determine important conformations, the peptide needs to be limited to a single conformational space region, and then it is determined whether this is a useful form. It is possible to determine which conformation is biologically important by inspecting many conformations.

The multi-disulfide-bond long-chain peptide can be derived from Achyranthes bidentata Blume, or Amaranthaceae and Achyranthes L. plants.

The multi-disulfide-bond long-chain peptide can be obtained by extraction, separation and purification, organic synthesis or genetic engineering in biological materials.

There are new methods for generating more accurate synthetic structures. It should be considered that the methods should maintain certain flexibility. That is, if a designed structure is too rigid, other structures with desirable characteristics for in vivo biological activity may not be adopted, considering that the adjustment can be achieved by a slightly flexible structure. A valuable understanding for necessary conditions for peptide receptors, enzyme active sites, and diverse other biological processes is obtained through techniques and methods suitable for the design of synthetic peptide.

The properties of the peptide in the biological system depend on the structure. Therefore, the capability to use reasonable design to produce useful peptide depends on the corresponding capability to determine the specific relationships between the molecular structures and biological activity. The capability to identify these relationships is determined by various uncertainties, both in biological test systems and in data interpretation. A more complicated factor is the difficulty in determining the three-dimensional structure of the peptide. Many peptides are inherently flexible and thus adopt many conformations in solutions. The problem is to determine one of the conformations responsible for the observed peptide activity, and many peptides can be active in more than one conformation. The use of conformational constraints is useful to clarify these structure-function relationships. If the peptide is limited to a specific conformation or closely related to a family with an active conformation, then the measured activity reflects the structure. Although the molecules cannot be completely rigid, it is also possible to attribute certain biological activities to the structure of the constituent cause by generating analogs with designated structural motifs.

The objective of the present invention is a multi-disulfide-bond long-chain peptide with neuroprotective activity, characterized in that the amino acid sequence of polypeptide comprises the following features, or ABPPk2, or having certain three-dimensional spatial structure:
(1) The multi-disulfide-bond long-chain peptide with y is characterized in that the amino acid sequence of polypeptide is: Cys-X1-Ser-Cys-X2-Gln-His-Asn-Cys-Cys-Ser-Gly-Val-Cys-Val-X3-Phe-Tyr-Gly-Val-Cy s-Tyr wherein
   X1 is a tetrapeptide, pentapeptide or hexapeptide sequence connected by 4-6 amino acids successively, and the amino acids are selected from 1-6 of Ser, Gly, Leu, Lys and Thr;
   X2 is a tetrapeptide, pentapeptide or hexapeptide sequence connected by 4-6 amino acids successively, and the amino acids are selected from 1-5 of Gly, Ala, Ile, Arg and Pro;
   X3 is a tetrapeptide, pentapeptide or hexapeptide sequence connected by 4-6 amino acids successively, and the amino acids are selected from 1-5 of Thr, Leu, Ile, Pro and Val.
(2) Of these peptides of neuroprotective activity is a key polypeptide termed as ABPPk2. The sequence of ABPPk2 is Cys-Leu-Glu-Ser-Gly-Thr-Ser-Cys-Ile-Pro-Gly-Ala-Gln-His-Asn-Cys-Cys-Ser-Gly-Val-C ys-Val-Pro-Ile-Val-Thr-Ile-Phe-Tyr-Gly-Val-Cys-Tyr
   (i.e., CLESGTSCIPGAQHNCCSGVCVPIVTIFYGVCY).
(3) Three pairs of disulfide bonds are connected in CysI-CysIV (that is, the sulfur in the first Cys is connected with the sulfur in the fourth Cys to form a disulfide bond), CysII-CysV (that is, the sulfur in the second Cys is connected with the sulfur in the fifth Cys to form a disulfide bond), and CysIII-CysVI (that is, the sulfur in the third Cys is connected with the sulfur in the sixth Cys to form a disulfide bond).
(4) A three-dimensional spatial structure of the multi-disulfide-bond long-chain peptide comprises two or three pairs of β-antiparallel structures which are located at N-terminal, a middle sequence and C-terminal of the multi-disulfide-bond long-chain peptide.
(5) Interaction sites between the ABPPk2 and NMDA2B receptor are reserved, i.e., Gln13, Asn15, Phe28 and Tyr33.
(6) The peptide comprises a linear peptide formed by an amino group and a carbonyl group at the N-terminal and the C-terminal of the peptide, or a cyclic peptide formed by peptide bonds between the amino group and the carbonyl group at the N-terminal and the C-terminal of the peptide.
(7) A disulfide covalent bond in the multi-disulfide-bond long-chain peptide comprises groups X1, X2 and X3 and cysteines in the fixed sequence in addition to the groups; and when two cysteines are separated by more than nine amino acid residues, the corresponding cysteines form the disulfide covalent bond.
(8) A glycosidic bond in the multi-disulfide-bond long-chain peptide comprises groups X1, X2 and X3, and side chain amino groups (Lys and Arg), carbonyl groups (Glu and Asp) and amide groups (Gln and Asn) in the fixed sequence in addition to the groups; the amino groups, the carbonyl groups or the amide groups can form glycosidic bonds with the following monosaccharides or disaccharides; the monosaccharides comprise one or more than one of arabinose, ribose, xylose, lyxose, glucose, mannose, fructose and galactose; and the disaccharides comprise one or more than one of maltose, lactose, sucrose, trehalose, cellobiose, gentiobiose and melibiose.
(9) The N-terminal in the multi-disulfide-bond long-chain peptide is covalently linked to a pyroglutamic acid or acetyl group, and the acetyl group comprises one or more than one of acetylcholine, paracetamol, acetylsalicylic acid, acetic acid, acetamide and acetic anhydride.

Another aspect of the present invention is a method for neuroprotection, wherein a pharmaceutical composition comprising an effective amount of at least one multi-disulfide-bond long-chain peptide of the present invention is administered to an individual which needs the pharmaceutical composition. A pharmaceutical composition comprising at least one multi-disulfide-bond long-chain peptide of the present invention and a pharmaceutically acceptable excipient or carrier is also the objective of the present invention.

### Description of Drawings

Fig. 1 shows a purification process of ABPPk2 in an embodiment. (a) Primary separation of preparative column; (b) re-separation of analytical column; (c) separation of ABPPk2 monomer by diol HILIC column.
Fig. 2 is an amino acid sequence diagram of Edman degradation of ABPPk2 in an embodiment.
Fig. 3 is a tandem mass spectrum of ABPPk2 in an embodiment. (a) IAM derivative of ABPPk2; (b) HCD fragmentation diagram of ABPPk2; (c) secondary mass spectrum of HCD fragmentation of ABPPk2 after acid hydrolysis for 5 min.
Fig. 4 is a primary amino acid sequence diagram of ABPPk2 in an embodiment; (A) Edman degradation data, (B) HCD data of ABPPk2 IAM derivative, (C) HCD data of ABPPk2, and (D) data of ABPPk2 after acid hydrolysis for 5 min.
Fig. 5 shows a connection mode of disulfide bonds of ABPPk2 in an embodiment.
Fig. 6 is a three-dimensional spatial structural diagram of ABPPk2 in an embodiment.
Fig. 7a shows binding three-dimensional conformation of ABPPk2 and NMDA2B (docking score 1249.709).
Fig. 7b shows electrostatic interaction between ABPPk2 and NMDA2B (A. electrostatic surface of NMDA2B; B. electrostatic surface of ABPPk2; and C. electrostatic surface of complex ABPPk2&NMDA2B).
Fig. 7c shows a binding mode of ABPPk2 and NMDA2B.
Fig. 8a shows the effect of different concentrations of NMDA on the activity of cultured hippocampal neurons in an embodiment (x±SD, n=8). ^{∗}Compared with the normal control group, p<0.05; #compared with the normal control group, p<0.01;
Fig. 8b shows the effect of NMDA that acts for different times on the activity of cultured hippocampal neurons in an embodiment (x±SD, n=8). ^{∗}Compared with the normal control group, p<0.05;
Fig. 8c shows the effect of different concentrations of ABPPk2 on hippocampal neuron damage induced by NMDA in an embodiment (x±SD, n=8). ^{∗}Compared with the NMDA damage control group, p<0.05; *compared with the NMDA damage control group, p<0.01.
Fig. 8d shows the effect of MK801 on hippocampal neuron damage induced by NMDA in an embodiment (x±SD, n=8). ^{∗}Compared with the NMDA damage control group, p<0.05; ^{∗}compared with the NMDA damage control group, p<0.01.
Fig. 8e shows the effect of ABPPk2 on cultured hippocampal neuron damage induced by NMDA in an embodiment (x±SD, n=8). ^{∗}Compared with the NMDA damage control group, p<0.05; *compared with the NMDA damage control group, p<0.01.
Fig. 8f shows the effect of ABPPk2 on cultured hippocampal neuron damage induced by NMDA in an embodiment (x±SD, n=8). ^{∗}Compared with the NMDA damage control group, p<0.01.
Fig. 9 shows cortical neurons after OGD damage and treatment with different drugs observed under a light microscope in an embodiment. (A. Normal neurons; B. OGD damage for 2h; C. positive control group; D. ABPPk2 low-dose group; E. ABPPk2 medium-dose group; F. ABPPk2 high-dose group, x20).
Fig. 10 shows the effect of ABPPk2 on activity of cortical neurons after OGD damage in an embodiment.
Fig. 11 shows the effect of ABPPk2 on apoptotic bodies of cortical neurons after OGD damage in an embodiment.
Fig. 12 shows the effect of ABPPk2 on apoptosis of cortical neurons after OGD damage in an embodiment.
Fig. 13 shows the effect of ABPPk2 on mitochondrial membrane potential of cortical neurons after OGD damage in an embodiment.
Fig. 14 shows the effect of ABPPk2 on ROS content of cortical neurons after OGD damage in an embodiment.
Fig. 15 shows the effect of ABPPk2 on expression of Bcl-2 and Bax proteins of cortical neurons after OGD damage in an embodiment; the left columns of the histograms represent Bcl-2, and the right columns represent Bax.
Fig. 16 shows the effect of ABPPk2 on expression of Cleaved caspase-3 protein of cortical neurons after OGD damage in an embodiment.
Fig. 17 shows the effect of ABPPk2 on Cyt C expression in mitochondria and cytoplasma of cortical neurons after OGD damage in an embodiment; the left columns of the histograms represent cytoplasma, and the right columns represent mitochondria.
Fig. 18 shows the effect of ABPPk2 on AIF expression in mitochondria and nucleus of cortical neurons after OGD damage in an embodiment; the left columns of the histograms represent nucleus, and the right columns represent mitochondria.
Fig. 19 shows the effect of ABPPk2 on p-AKT expression of cortical neurons with OGD damage after treatment with PI3K inhibitor in an embodiment.
Fig. 20 shows the effect of ABPPk2 on cleaved caspase-3 expression of cortical neurons with OGD damage after treatment with PI3K inhibitor in an embodiment.
Fig. 21 shows the effect of ABPPk2 on growth of primary fetal rat hippocampal neurons in an embodiment.
Fig. 22 shows the effect of ABPPk2 on growth of primary fetal rat hippocampal neurons observed in a live cell station in an embodiment.
Fig. 23 shows the effect of treatment with ABPPk2 for 24h on neurite growth cone and filopodia of hippocampal neurons in an embodiment.
Fig. 24 shows the effect of treatment with ABPPk2 for 24h on neurite growth of hippocampal neurons in an embodiment.
Fig. 25 shows the effect of treatment with ABPPk2 for 48h on neurite growth of hippocampal neurons in an embodiment.
Fig. 26 shows the effect of ABPPk2 on synapse formation of hippocampal neurons in an embodiment.
Fig. 27 shows a three-dimensional spatial structure of a multi-disulfide-bond long-chain peptide after homology modeling in an embodiment.

### Detailed Description

For the convenience of understanding the present invention, the present invention will be described below more comprehensively with reference to relevant drawings. Preferred embodiments of the present invention are given in the drawings. However, the present invention may be achieved in many different forms and will not be limited to the embodiments described herein. On the contrary, the purpose of providing the embodiments is to understand the disclosure in the present invention more thoroughly and comprehensively.

Unless otherwise defined, all technical and scientific terms used herein have the same meanings as those generally understood by those skilled in the art in the present invention. The terms used in the description of the present invention herein are intended to merely describe concrete embodiments, not to limit the present invention. The term "and/or" used herein comprises any and all of combinations of one or a plurality of related listed items.

Specific embodiments are provided below:

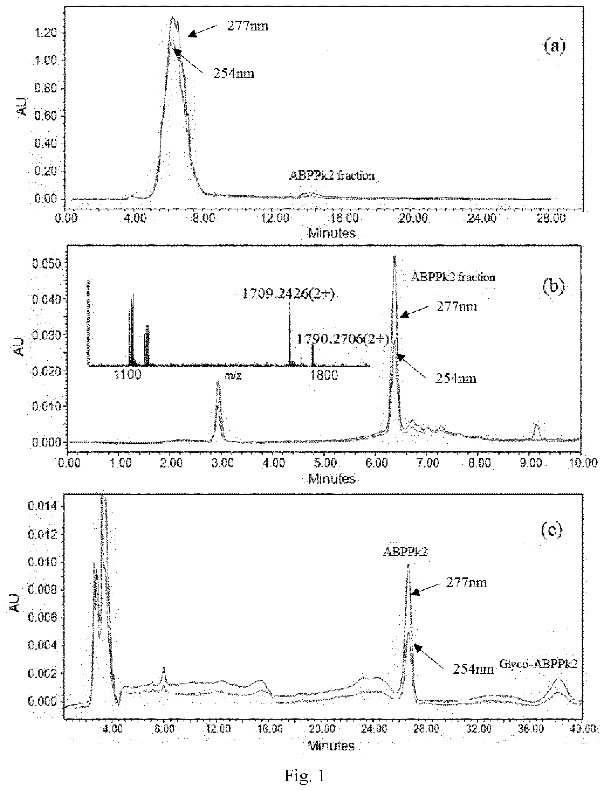

Embodiment 1: Extraction, separation and purification process of ABPPk2 (1) Extraction of ABPPk2:
Single medicine Achyranthes bidentata Blume decoction pieces were extracted with an aqueous solution for three times, and the extracts were combined to obtain the Achyranthes bidentata Blume aqueous solution. The Achyranthes bidentata Blume aqueous solution was added with ammonium sulfate to a mass concentration of 50% at 4°C, and centrifuged at 15000rpm for half an hour; and the supernatant after centrifugation was continuously added with ammonium sulfate to a mass concentration of 80% at 4°C, and centrifuged at 15000rpm for half an hour; the precipitate was moved to a dialysis bag with a minimum molecular weight cut-off of 1000, and dialyzed with pure water; the sample in the dialysis bag was retained; and the sample was freeze-dried to obtain a crude extract of Achyranthes bidentata Blume.
(2) Enrichment of ABPPk2:
   The crude extract of Achyranthes bidentata Blume was dissolved according to the ratio that 2g of the crude extract of Achyranthes bidentata Blume was dissolved in 100 mL of water, and Sevage reagent was added in a volume ratio of Sevage reagent: sample=1:3 by Sevage reagent method (composition: chloroform: n-butanol (volume ratio)=5:1). The water layer was extracted twice and the intermediate solid layers were combined.
(3) Separation of ABPPk2:
   Primary separation of ABPPk2 was conducted using a preparative C18 chromatographic column (Innoval ODS-P, 5µm, 100 Å, 21.2×250mm, Agela Technologies) by taking a methanol aqueous solution of 70% volume ratio (containing 0.5% formic acid) as a mobile phase with detection wavelengths of 254nm and 277nm and flow rate of 10mL/min. The retention time was about 14min. After receiving according to the retention time, the solutions were combined and concentrated. The primarily separated polypeptide after concentrated was purified again using an analytical C18 chromatographic column (Cosmosil, 5 µm, 4.6×250 mm) with a binary mobile phase. Mobile phase A was an aqueous solution containing 0.5% formic acid and mobile phase B was acetonitrile. The elution gradient was 0-8 min, 30-70% B, 8.1-9 min, 100% B, 9.1-10 min, 30% B; the flow rate was 1 mL/min; and the retention time was about 6.5 min. After receiving according to the retention time, the solutions were combined and concentrated. Monomer separation of ABPPk2 was conducted using diol HILIC column (YMC-Pack Diol-NP, 5 µm, 10×250 mm); the mobile phase was 80:20 acetonitrile: 200mM ammonium acetate solution; the flow rate was 4mL/min; and the retention time was about 26min. After receiving according to the retention time, the solutions were combined, concentrated and desalted. The above separation results are shown in Fig. 1.

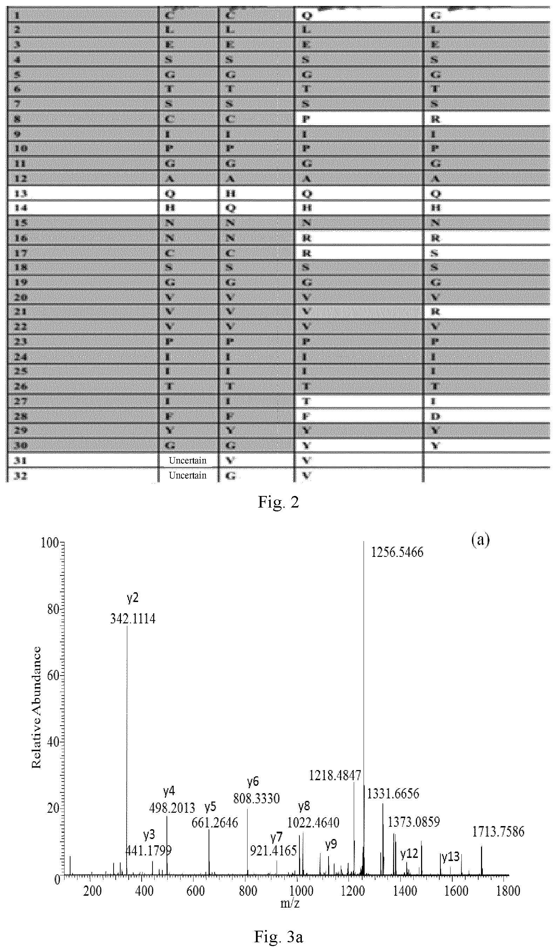

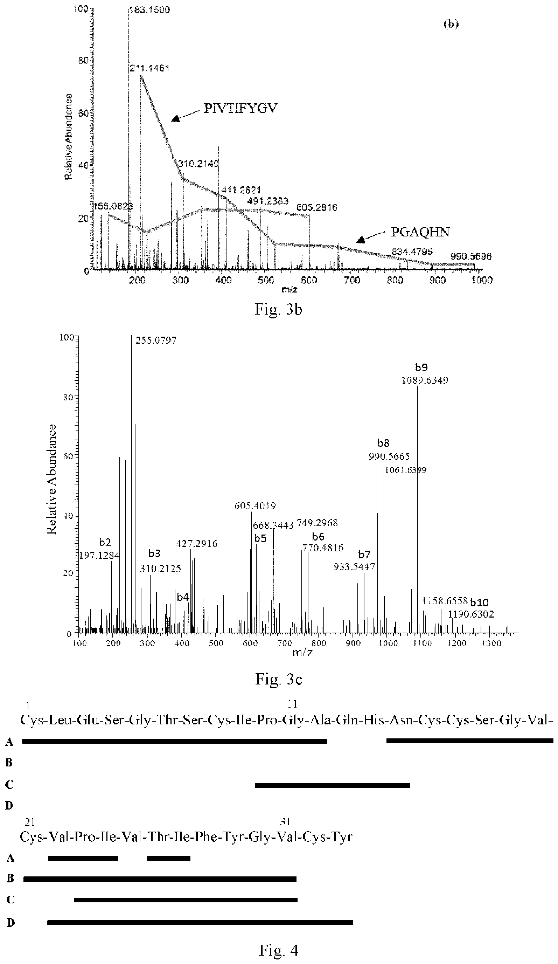

Embodiment 2: Amino acid sequence analysis of ABPPk2
(1) Edman degradation analysis of amino acid sequence of ABPPk2:
   The Edman degradation experiment of ABPPk2 was carried out on a PPSQ-51A system 200V (Shimadzu, Japan) sequencer, and the amino acid sequence results obtained were shown in Fig. 2.
(2) Mass spectrometry and primary amino acid sequence of ABPPk2:
   In a positive ion mode, high resolution mass spectrometry Thermo-Orbitrap-Elite is used to analyze the primary amino acid sequence of ABPPk2. The classic derivatization reagent DTT/IAM is used for the reduction and derivatization of cysteine in the disulfide bond of ABPPk2. After the ABPPk2 was fully derivatized, the mass number of each disulfide bond was increased by 2×58.0293. Fig. 3a is an HCD secondary mass spectrum of an IAM derivative of ABPPk2. A peptide sequence CVPIVTIFYGVCY can be determined, as shown in Table 1. It should be noted that leucine L and isoleucine I were determined by Edman degradation. CY sequence of C-terminal was determined by mass spectrometric data after acid hydrolysis of 5min. Fig. 3b is an HCD secondary mass spectrum of ABPPk2. Two peptide sequences PIVTIFYGV and PGAQHN can be determined, as shown in Table 2. Fig. 3c is an HCD secondary mass spectrum of a certain peptide in LC-MS analysis when the ABPPk2 is subjected to acid hydrolysis for 5 min. The sequence of the peptide can be determined as VPIVTIFYGVCY, as shown in Table 3. By combining Edman degradation with mass spectrometry, the primary amino acid sequence of the ABPPk2 is obtained as CLESGTSCIPGAQHNCCSGVCVPIVTIFYGVCY (as shown in Fig. 4, containing three pairs of disulfide bonds).

Information of ABPPk2 SEQ ID No.1:
(a) Sequence features
Length: 33 amino acids
Type: amino acid
Chain type: single chain

(b) Molecular type: polypeptide
Feature name keywords: DISULFID; DOMAIN; STRAND
Original source: Achyranthes bidentata Blume

**Table 1. Amino Acid Sequence Information Deduced from HCD Secondary Mass Spectrum of IAM Derivative of ABPPk2**

| y-ion series composition | Experimental mass-to-charge ratio *m*/*z* | Theoretical mass-to-charge ratio *m*/*z* |
|---|---|---|
| CY | 342.1114 | 342.1124 |
| VCY | 441.1799 | 441.1808 |
| GVCY | 498.2022 | 498.2022 |
| YGVCY | 661.2646 | 661.2656 |
| FYGVCY | 808.3330 | 808.3340 |
| IFYGVCY | 921.4165 | 921.4180 |
| TIFYGVCY | 1022.4640 | 1022.4657 |
| VTIFYGVYC | 1121.5313 | 1121.5341 |
| IVTIFYGVCY | 1234.6171 | 1234.6182 |
| PIVTIFYGVCY | 1331.6656 | 1331.6710 |
| VPIVTIFYGVCY | 1430.7327 | 1430.7394 |
| CVPIVTIFYGVCY | 1590.7622 | 1590.7700 |

**Table 2. Amino Acid Sequence Information Deduced from HCD Secondary Mass Spectrum of ABPPk2**

| Ion series composition | Experimental mass-to-charge ratio *m*/*z* | Theoretical mass-to-charge ratio *m*/*z* |
|---|---|---|
| PI | 211.1451 | 211.1447 |
| PIV | 310.2140 | 310.2131 |
| PIVT | 411.2621 | 411.2607 |
| PIVTI | 524.3465 | 524.3448 |
| PIVTIF | 671.4155 | 671.4132 |
| PIVTIFY | 834.4795 | 834.4766 |
| PIVTIFYG | 891.5008 | 891.4980 |
| PIVTIFYGV | 990.5696 | 990.5664 |
| PG | 155.0823 | 155.0821 |
| PGA | 226.1197 | 226.1192 |
| PGAQ | 354.1789 | 354.1777 |
| PGAQH | 491.2383 | 491.2367 |
| PGAQHN | 605.2816 | 605.2796 |

**Table 3. Amino Acid Sequence Information Deduced from Secondary Mass Spectrum of Acid Hydrolysis Product of ABPPk2**

| Ion series composition | Experimental mass-to-charge ratio *m*/*z* | Theoretical mass-to-charge ratio m/z |
|---|---|---|
| VP | 197.1284 | 197.1290 |
| VPI | 310.2125 | 310.2131 |
| VPIV | 409.2809 | 409.2815 |
| VPIVTI | 623.4128 | 623.4132 |
| VPIVTIF | 770.4816 | 770.4816 |
| VPIVTIFY | 933.5447 | 933.5450 |
| VPIVTIFYG | 990.5665 | 990.5664 |
| VPIVTIFYGV | 1089.6349 | 1089.6349 |
| VPIVTIFYGVC-[H_{2]} | 1190.6302 | 1190.6284 |
| VPIVTIFYGVC-[H₂S] | 1158.6558 | 1158.6563 |
| VPIVTIFYGVCY-[H_{2]} | 686.3555²⁺ | 686.3545²⁺ |

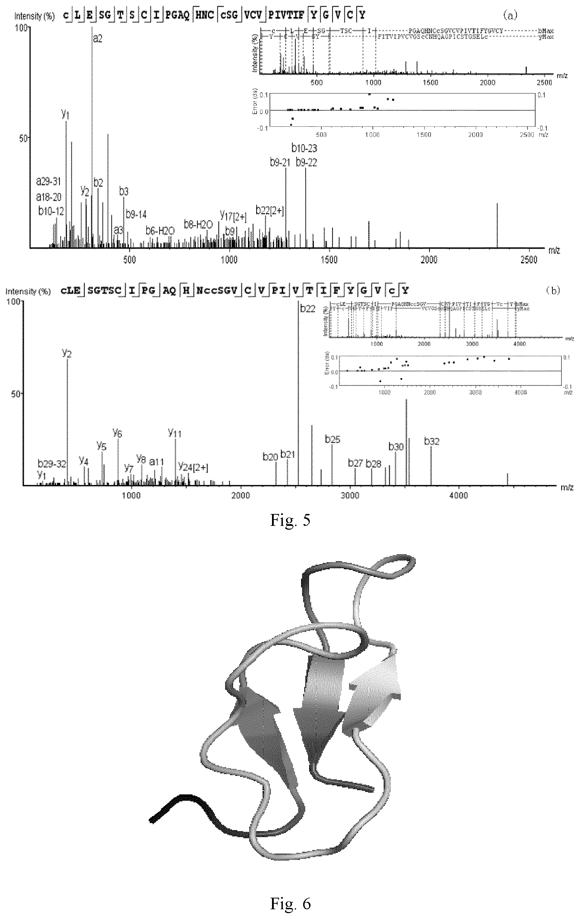

Embodiment 3: Connection mode of disulfide bonds of ABPPk2:
A TCEP partial reduction method is used for ABPPk2 to obtain the connection mode of the disulfide bonds. Namely, at room temperature, TCEP is used to partially reduce the disulfide bonds of ABPPk2, and NEM is used for derivatization. Finally, TCEP reduces all the disulfide bonds to sulfydryl at 65°C. The mass spectrum fragmentation data of Orbitrap was imported into Peaks to search a database (Fig. 5). The fasta file was the primary sequence of ABPPk2. The connection modes of three pairs of disulfide bonds of ABPPk2 are Cys1-Cys17, Cys8-Cys21 and Cys16-Cys32 (Table 4), i.e., CysI-CysIV, CysII-CysV and CysIII-CysVI.

**Table 4. TCEP/NEM step reduction of ABPPk2 to obtain the connection mode information of the disulfide bonds**

| No. | Peptide | -10lgP | Mass | m/z | z | RT | ppm |
|---|---|---|---|---|---|---|---|
| 1 | C(+125.05)LESGTSCIPGAQHNCC(+125.05)S GVCVPIVTIFYGVCY | 16.06 | 3672.5 | 1225.2 | 3 | 3.71 | -0.7 |
| | | | 901 | 031 | | | |
| 2 | C(+125.05)LESGTSCIPGAQHNC(+125.05)C( +125.05)SGVCVPIVTIFYGVC(+125.05)Y | 55.55 | 3922.6 | 1308.5 | 3 | 3.85 | 0.3 |
| | | | 865 | 698 | | | |

Embodiment 4: Three-dimensional spatial structure of ABPPk2:
The three-dimensional spatial structure of ABPPk2 was obtained by two-dimensional NMR technology, as shown in Fig. 6. The experimental instrument is a Bruker 850MHz NMR spectrometer equipped with a low temperature probe. The sample preparation process is: about 2 mg of ABPPk2 was dissolved in 1 mL of sodium phosphate buffer solution (50 mM), and the solvents were water: heavy water: deuterated acetonitrile (volume ratio) according to 6:1:3. Spectra collected at room temperature include NOESY, TOCSY, DQF-COSY, 1H-13C HSQC and 1H-15N HSQC. As shown in the figure, the ABPPk2 is based on loop structure and includes three anti-parallel β-sheet structures located in Glu3-Ile9, Gly19-Pro23 and Phe28-Cys32.

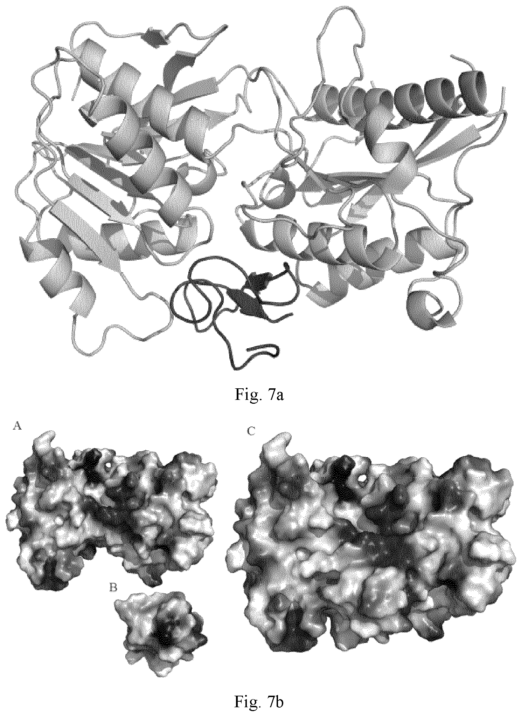

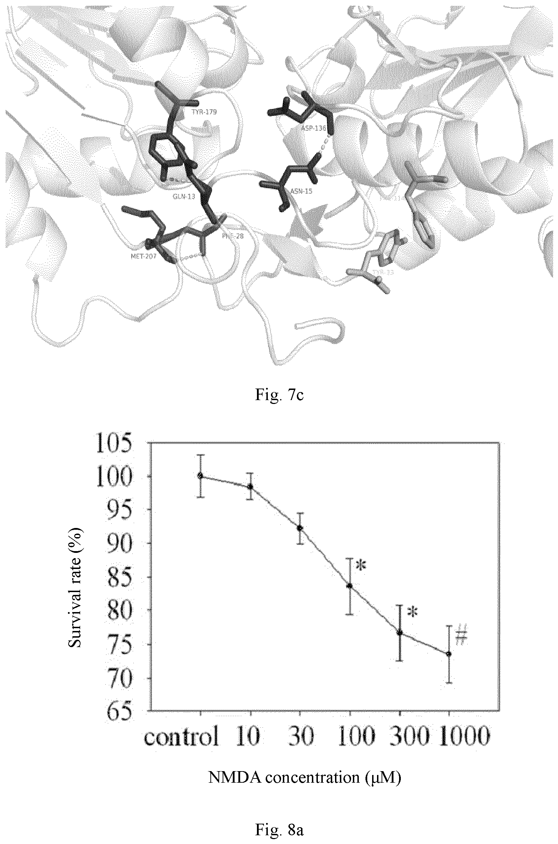

Embodiment 5: Research on the interaction between ABPPk2 and NMDA2B receptor:
Protein-protein molecular docking was conducted by ZDOCK tool, and scoring and sorting can be conducted according to different protein-protein binding conformations. The protein-protein binding three-dimensional conformation of ABPPk2 and NMDA2B is shown in Fig. 7a. Small protein can fit well in the NMDA2B inhibitor ligand pocket according to the complex of ABPPk2 and NMDA2B receptor, and the two have good shape complementarity. Electrostatic interaction is one of the most important acting forces of protein-protein binding. The electrostatic interaction between ABPPk2 and NMDA2B is shown in Fig. 7b. In the figure, A represents the electrostatic surface of NMDA2B, B is the electrostatic surface of ABPPk2 small protein, and C is the electrostatic surface of the complex of ABPPk2 and NMDA2B. It can be seen from the figure that the contact surfaces of the two proteins can be combined through the electrostatic interaction. Especially, the positively charged group on the front end of ABP and the negatively charged group on the right side have opposite charges to the contact part of the NMDA2B protein, which can form a good electrostatic effect. Fig. 7c shows the interaction modes of hydrogen bonds of the two proteins. It can be seen from the figure that the two proteins form 3 pairs of hydrogen bonds (Gln13, Asn15 and Phe28 of ABPPk2 correspond to Tyr179, Asp136 and Met207 of NMDA2B respectively). In addition, Tyr33 of ABPPk2 can form a π-π interaction with Phe114. Therefore, the structural domain of the NMDA2B receptor that interacts with ABPPk2 is located in the N-terminal ATD region, and the key residues of ABPPk2 are Gln13, Asn15, Phe28 and Tyr33.

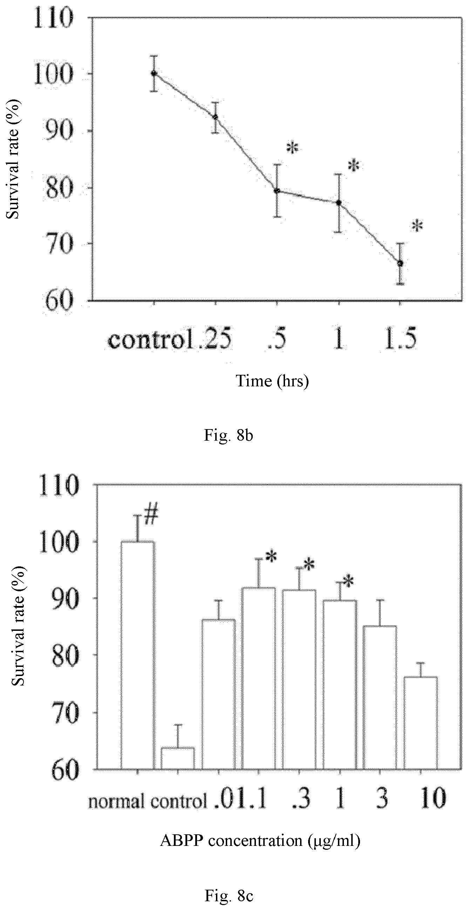

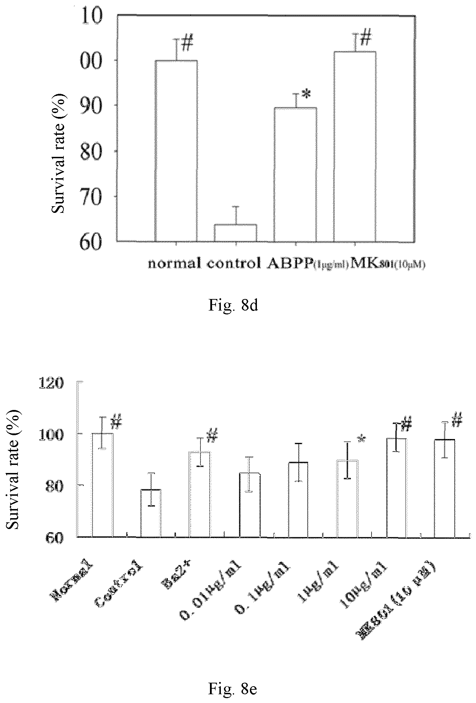

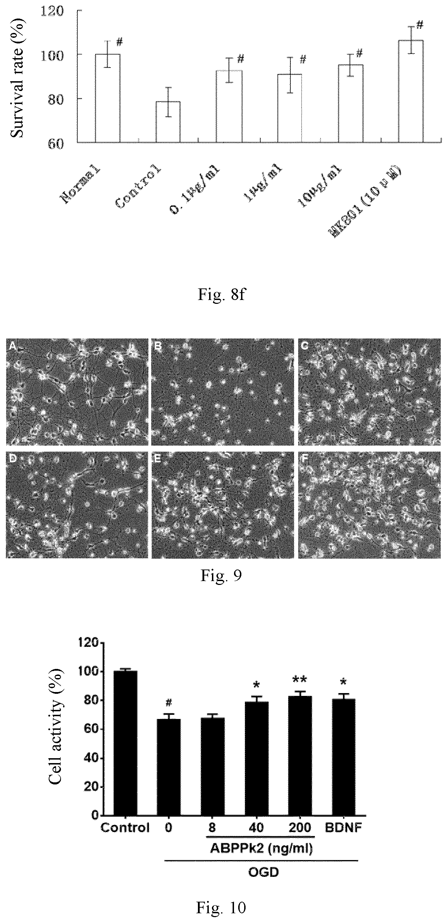

Embodiment 6: Effect of ABPPk2 on NMDA damage of primary cultured hippocampal neurons:
(1) Establishment of NMDA damage model. Primary culture of hippocampal neurons: SD (Sprague-Dawley) female rats pregnant for 18-19 days were anesthetized with a compound anaesthetic agent; fetal rat brains were taken under aseptic conditions and placed in HBSS solution (8.4g/L of NaCl, 0.224g/L of KCl, 2.38g/L of HEPES, 500,000 units of penicillin sodium and 500,000 units of kanamycin sulfate; the pH was adjusted to 7.1-7.2 with 1M NaOH; then the osmotic pressure was adjusted to about 290±10m Osm with ultrapure water; and after filtration, the solution was stored at 4°C); the meninges were removed under a stereoscopic dissecting microscope; the bilateral hippocampus was removed and digested in 0.125% pancreatic protease at 37°C for 10 min; and complete media (90% DMEM medium +10% FBS) (FBS: fetal bovine serum) was used to terminate the digestion; the bilateral hippocampus was washed for 3 times with the complete mediato make a single cell suspension; the cell density was adjusted to about 7-8×10⁵ /ml; and the bilateral hippocampus was inoculated to a 6-well/96-well cell culture plate/60mm culture dish/slide coated with L-polylysine. After 24 hours, the media were changed to serum-free neurobasal media (GIBCO) which were then placed in a 5% CO₂, 37°C incubator for culture; and half of the solution was changed every three days. Cultivation was conducted until a rich neural network was formed, which indicated that the neurons were mature.
(2) NMDA damage treatment of hippocampal neurons: ① The original media were sucked out and the cells were washed with magnesium-free extracellular fluid for three times, each for 5 minutes. ② 10mM NMDA (N-methyl-D-aspartic acid) and 10mM Gly (glycine) were prepared with magnesium-free extracellular fluid, and NMDA was prepared to different concentrations with the magnesium-free extracellular fluid, wherein the final concentration of Gly was 10µM. The magnesium-free extracellular fluid of the normal control group does not contain NMDA and Gly, and the rest is the same as the experimental group. After damage, the cells were washed for three times with DMEM medium, each for 5 minutes. ③ The original culture media were re-added to continue for culturing for 24 hours.
(3) Detection of viability of hippocampal neurons after NMDA damage: ① The hippocampal neurons were inoculated into a 96-well culture plate at 8×10⁵ cells/ml, 0.1ml per well, andcultured in a 5% CO₂ incubator for 7-8 days. ② After NMDA damage treatment and continuous culture for 24 hours, the culture solution was sucked off. ③ 100µl of DMEM culture medium containing MTT (3-(4,5-dimethylthiazole-2)-2,5-diphenyltetrazolium bromide, 0.5mg/ml) was added to each well, and the culture plate was put into 37°C, 5% CO₂ incubator for culturing for 4h. ④ 100µl of 20% SDS was added to each well (20g of sodium lauryl sulfate was dissolved in 50ml of ddH₂O+50ml of N,N dimethylformamide) at 37°C for 20 h. ⑤ The solution was shaken on a horizontal shaker for 5 min. ⑥ The optical density (OD) was measured at 570nm (reference wavelength of 490nm) on a microplate reader.
(4) Effect of different concentrations of NMDA acting for 1 hour on activity of hippocampal neurons. The experiment was divided into 6 experimental groups: a normal group, 10µM NMDA group, 30µM NMDA group, 100µM NMDA group, 300µM NMDA group and 1000µM NMDA group. The rest is the same as above. The results showed that compared with the normal control group, with the increase of NMDA concentration, the activity of hippocampal neurons was decreased significantly in a dose-dependent manner. The results were shown in Fig. 8a.
(5) Effect of NMDA (100µM) acting for different time on activity of hippocampal neurons. The experiment was divided into 5 experimental groups: a normal group, NMDA (100µM) 15-min group, NMDA (100µM) 30-min group, NMDA (100µM) 60-min group and NMDA (100µM) 90-min group. The rest is the same as above. The results showed that with the increase of 100µM NMDA damage time, the activity of the hippocampal neurons was gradually decreased. The results were shown in Fig. 8b. In subsequent experiments, NMDA (100µM) 30min was selected as the NMDA damage model.
(6) Effect of ABPPk2 on decrease of hippocampal neuron activity caused by NMDA. Effect of different concentrations of ABPPk2 on hippocampal neuron damage caused by NMDA (100µM, 30min). The experiment was divided into 9 experimental groups: a normal group, NMDA (100µM, 30min) group (as a control group), ABPPk2 (0.01µg/ml) group, ABPPk2 (0.1µg/ml) group, ABPPk2 (0.3µg/ml) group, ABPPk2 (1µg/ml) group, ABPPk2 (3µg/ml) group, ABPPk2 (10µg/ml) group and MK801 (dizocilpine, 10µM) group (as a positive control group). Three administration ways were adopted for ABPPk2: ① ABPPk2 was administered 12 hours before and during the NMDA damage. ② ABPPk2 was administered during NMDA damage. ③ ABPPk2 was administered during 24 hours of culture after NMDA damage.
(7) Effect of pre-administration of ABPPk2 on the damage of cultured hippocampal neurons caused by NMDA. The effect of ABPPk2 on the damage of cultured hippocampal neurons caused by NMDA was observed by MTT method. Before NMDA damage, after culture for 12 hours using serum-free neuron culture medium containing different concentrations of ABPPk2 (0.01-10µg/ml) or serum-free neuron culture medium containing MK801 (10µM), NMDA (100µM) damaged neurons for 0.5 h (containing the corresponding different concentrations of ABPPk2 (0.01-10µg/ml) or MK801 (10µM) for each group during damage); and after continuous culture in the serum-free neuron medium for 24 hours, MTT detection was conducted and cell activity of each group was observed. The results showed: in the NMDA damage group, the hippocampal neuron activity was significantly decreased, and the NMDA damage group was used as the control group; compared with the NMDA damage group, the hippocampal neuron activity in the normal group was significantly increased (p<0.01). The effect of ABPPk2 on the damage of the cultured hippocampal neurons caused by NMDA was concentration-dependent. The ABPPk2 0.1µg/ml group, the ABPPk2 0.3µg/ml group, and the ABPPk2 1µg/ml group can effectively reduce the decrease of hippocampal neuron activity caused by NMDA damage. The results were shown in Fig. 8c. The MK801 (10µM) group as the positive control group can effectively reduce the decrease of hippocampal neuron activity caused by NMDA damage. The results were shown in Fig. 8d.
(8) Effect of simultaneous administration of ABPPk2 on the damage of cultured hippocampal neurons caused by NMDA. The effect of ABPPk2 on the damage of cultured hippocampal neurons caused by NMDA was observed by MTT method. NMDA (100µM) damaged neurons for 0.5 h (containing different concentrations of ABPPk2 (0.01-10µg/ml) or MK801 (10µM) during damage); and after continuous culture in the original culture medium for 24 hours, MTT detection was conducted and cell activity of each group was observed. The results showed: in the NMDA damage group, the hippocampal neuron activity was significantly decreased, and the NMDA damage group was used as the control group; compared with the NMDA damage group, the hippocampal neuron activity in the normal group was significantly increased (p<0.01). The effect of ABPPk2 on the damage of the cultured hippocampal neurons caused by NMDA was concentration-dependent. The ABPPk2 1µg/ml group and the ABPPk2 10µg/ml group can effectively reduce the decrease of hippocampal neuron activity caused by NMDA damage. The results were shown in Fig. 7. The MK801 (10µM) group as the positive control group can effectively reduce the decrease of hippocampal neuron activity caused by NMDA damage. On the other hand, Ba²⁺ in barium ions that replace calcium ions in the magnesium-free extracellular fluid during damage can effectively inhibit the damage of the cultured hippocampal neurons caused by NMDA (p<0.01). The results were shown in Fig. 8e.
(9) Effect of administration of ABPPk2 after NMDA damage on the damage of cultured hippocampal neurons caused by NMDA. The effect of ABPPk2 on the damage of cultured hippocampal neurons caused by NMDA was observed by MTT method. After NMDA (100µM) damaged neurons for 0.5 h, continuous culture was conducted in the original culture medium containing different concentrations of ABPPk2 (0.1-10µg/ml) or MK801 (10µM) for 24 hours; MTT detection was conducted and cell activity of each group was observed. The results showed: in the NMDA damage group, the hippocampal neuron activity was significantly decreased, and the NMDA damage group was used as the control group; compared with the NMDA damage group, the hippocampal neuron activity in the normal group was significantly increased (p<0.01). ABPPk2 can effectively reduce the decrease of hippocampal neuron activity caused by NMDA damage. The MK801 (10µM) group as the positive control group can effectively reduce the decrease of hippocampal neuron activity caused by NMDA damage. The results were shown in Fig. 8f.
(10) The results show that ABPPk2 has a protective effect on the damage of primary cultured hippocampal neurons induced by NMDA.

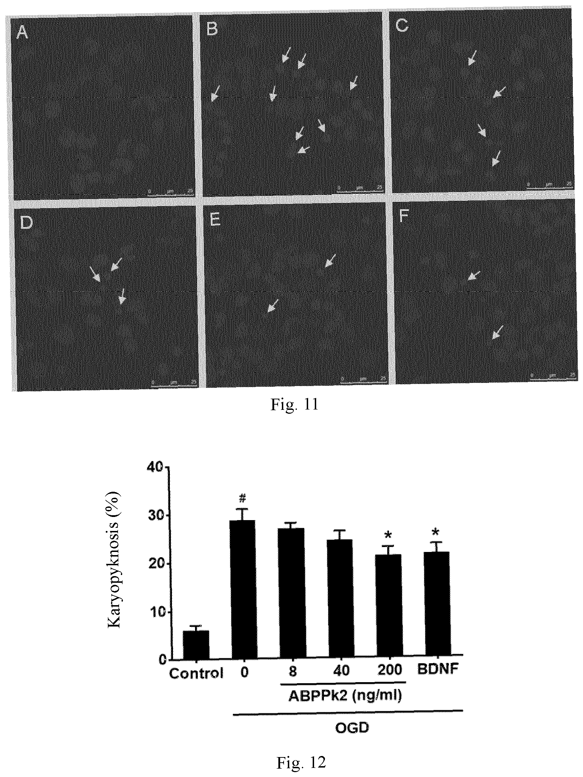

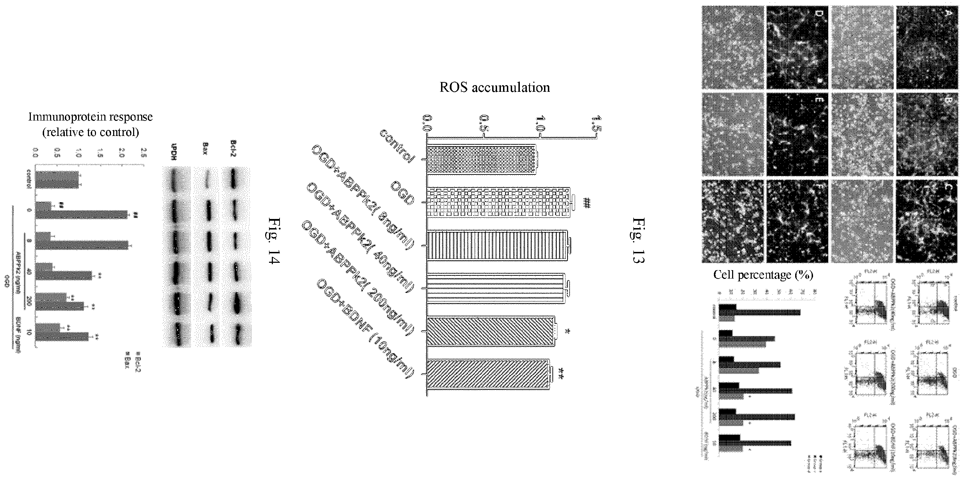

Embodiment 7: Protective effect of ABPPk2 on hypoxic-ischemic damage of primary cultured SD fetal rat cortical neurons:
(1) Normal culture of cortical neurons: embryos of SD rats pregnant for 17 days were taken to separate the cerebral cortex under a dissecting microscope, digest with 0.125% pancreatic protease at 37°C for 8 min, terminate the digestion with DMEM complete medium containing 10% fetal bovine serum (FBS), and gently blow to form a single cell suspension. The suspension was filtered with a 400-mesh screen, centrifuged at 1000 rpm for 5 min, and blown to form the single cell suspension. DMEM high glucose complete medium (containing 10% fetal bovine serum) was inoculated in a culture dish or culture plate pre-coated with polylysine (PLL) to culture in 5% CO₂ incubator with saturated humidity at 37°C. After the cells were cultured for 4 h to adhere to the wall, the cells were cultured with neuron culture medium (neurobasal+2% B27) for 3 d to observe the growth of neurons. The culture medium containing cytarabine (the final concentration of cytarabine was 2.5 µg/ml) was substituted with half quantity to culture for 3d to inhibit the growth of glial cells and miscellaneous cells to obtain purely cultured primary cortical nerve cells. The purity of the cortical neurons was identified by immunocytochemical staining as more than 90%.
(2) Oxygen glucose deprivation (OGD) damage model and drug treatment of cortical neurons
   After the cortical neurons were cultured to the 7th day, the original culture medium was sucked out, and the cells were rinsed twice with sugar-free DMEM basal medium, for 5 min each time. Each OGD damage group was added with DMEM sugar-free culture medium, placed in a Billups-Rothenberg anaerobic culture chamber, filled with 95% N₂/5% CO₂ to balance for 15 min, and sealed for 2 h to build an OGD model. After OGD, grouping and treatment were as follows: OGD damage group: the DMEM sugar-free culture medium was discarded and Neurobasal complete medium was added; OGD+ ABPPk2 low-dose group: the DMEM sugar-free culture medium was discarded and Neurobasal complete medium with ABPPk2 final concentration of 8 ng/ml was added; OGD+ ABPPk2 medium-dose group: the DMEM sugar-free culture medium was discarded and Neurobasal complete medium with ABPPk2 final concentration of 40 ng/ml was added; OGD+ ABPPk2 high-dose group: the DMEM sugar-free culture medium was discarded and Neurobasal complete medium with ABPPk2 final concentration of 200 ng/ml was added; OGD+ BDNF group: the DMEM sugar-free culture medium was discarded and Neurobasal complete medium with BDNF final concentration of 10 ng/ml was added as the positive control group. A normal control group was set; and the cells were cultured normally without any damage or drug treatment. The cells treated with drugs were put back into 37°C, 5% CO₂ incubator, reoxygenated and resugard for 24 h and subjected to subsequent experiments.
(3) Microscopic observation of cortical neurons after OGD damage and drug treatment: as shown in Fig. 9 (×20), A is a normal cortical neuron cell body with smooth surface and having networked intertwined protrusions, transparent cell bodies and obvious refractivity; B is an OGD damage group, having some pyknotic cell bodies, unobvious, reduced or vanished cell protrusions, decreased refractivity and some dead cells; C, E and F are a positive control group, ABPPk2 medium-dose treatment group and ABPPk2 high-dose treatment group; compared with the OGD damage group, some cell protrusions are also retracted, but the cell bodies were retracted a little, and largely adhered to the wall, and the number of dead cells is significantly reduced.
   Analysis of cortical neuron activity after OGD damage and drug treatment: cortical neurons were inoculated into 96-well plates at 1×10⁶ cells/ml; and 100µl cell suspension was added to each well. After normal culture to the 7th day, OGD damage and drug treatment were performed. After reoxygenation and resugaring for 24h, 100µl of Neurobasal medium containing 10µl of CCK-8 was added to each well to incubate in a 37°C, 5% CO₂ incubator for 2h. The absorbance (OD) value of the sample in each well was measured on the microplate reader at 450nm wavelength. Each experimental group included 10 complex holes, and the experiment was repeated for 3 times. The results were shown in Fig. 10: 2h after OGD damage, the cortical neuron activity was decreased significantly (compared with the normal control group, # P<0.01); ABPPk2 can effectively antagonize the decrease of cortical neuron activity caused by OGD damage. In the observed measurement range, as the dose of ABPPk2 was increased, its protective effect was more obvious (compared with the OGD damage group, ^{∗}P<0.05, ^{∗∗}P<0.01).
(4) Effect of ABPPk2 on apoptosis of cortical neurons in OGD damage: round slides pre-coated with PLL was put into a 24-well culture plate; cortical neurons were inoculated to the slides at a density of 1×10⁵ cells/ml; and 500µl of cell suspension was added to each well. After culturing to the 7th day, the grouping and treatment methods were the same as above. After reoxygenation and resugaring for 24 h, the culture solution was discarded, and PBS (IX, NaCl 8.0g, KCl 0.2g, Na₂HPO₄ 1.44g, KH₂PO₄ 0.24 g, and dd H₂O was added to 1000ml) was used for rinsing once. 300µl of 4% paraformaldehyde (PFA) was added to each well and fixed for 20min at room temperature; the fixing solution was discarded, and PBS (IX) was used for washing for 3 times, for 5min each time. 300µl of Hoechst 33258 working solution was added to each well, and stained for 15min in the dark at room temperature; the staining solution was discarded; and PBS (IX) was used for washing for 3 times, for 5min each time. The round slides were picked out one by one; the side inoculated with the cells was buckled down on the slide dripped with fluorescent mounting medium for mounting; and the slides were dried for 2 h in the dark at room temperature, and observed and photographed under a laser confocal microscope. Each group included 4 complex holes, and the experiment was repeated for 3 times. The results were shown in Fig. 11. A shows a cortical neuron in the normal control group. The nuclei are regular in shapes and sizes and mostly round, with almost no apoptotic bodies. B shows neurons after OGD damage; the nuclei are irregular in shapes and sizes, and are condensed, broken and chromatin condensed, and apoptotic bodies are increased; C, D, E and F respectively show neurons treated with low, medium, and high concentrations of ABPPk2 and positive control BDNF, and the shapes of the nuclei are improved, and the apoptotic bodies are reduced. The proportion of apoptotic cell nuclei to the total nuclei was calculated, and the results were shown in Fig. 12. Compared with the normal control group, the apoptotic bodies of cortical neuron nuclei after OGD damage were increased significantly (compared with the normal control group, # P<0.01). After treatment with different concentrations of ABPPk2, the apoptotic bodies of the cortical neuron nuclei were reduced (compared with OGD damage group, ^{∗}P<0.05, ^{∗∗}P<0.01), and dose-dependent, indicating that ABPPk2 can reduce the formation of the apoptotic bodies in the cortical neurons induced by OGD damage.
(5) Effect of ABPPk2 on mitochondrial membrane potential of cortical neurons after OGD damage: cortical neurons were inoculated in a 6-well plate at 1×10⁶ cells/ml; 2ml of cell suspension was added to each well; and the cortical neurons were cultured to the 7th day. The grouping and treatment methods were as described above. After reoxygenation and resugaring for 24h, the cell culture medium was discarded, placed on ice, and rinsed twice with PBS (IX). The mitochondrial membrane potential of the cortical neurons after OGD damage was detected by a JC-1 staining kit and a flow cytometer. The results were shown in Fig. 13. Treatment with ABPPk2 can alleviate the decline in mitochondrial membrane potential of the cortical neurons after OGD damage, and an obvious dose-effect relationship was presented. (The left figure is JC-1 staining, A. Control, B. OGD, C. ABPPk2 8ng/ml, D. ABPPk2 40ng/ml, E. ABPPk2 200ng/ml, F. BDNF 10ng/ml; and the right figure is the detecting result of the flow cytometer. Compared with the OGD damage group, ^{∗}P<0.05).
(6) Effect of ABPPk2 on ROS content of cortical neurons after OGD damage: cortical neurons were inoculated in a 6-well plate at 1×10⁶ cells/ml; 2ml of cell suspension was added to each well; and the cortical neurons were cultured to the 7th day. The grouping and treatment methods were as described above. After reoxygenation and resugaring for 24h, the cell culture medium was discarded, placed on ice, and rinsed twice with PBS (IX). The effect of ABPPk2 on the ROS content of the cortical neurons after OGD damage was detected by a reactive oxygen detection kit. The results were shown in Fig. 14. Treatment with ABPPk2 can reduce the ROS content of the cortical neurons after OGD damage, and an obvious dose-effect relationship was presented (compared with the normal control group, # P<0.01; and compared with the OGD damage group, ^{∗}P<0.05).
(7) Effect of ABPPk2 on expression of apoptosis-related protein in cortical neurons after OGD damage.

Extraction of total cell protein: the cortical neurons were inoculated in a 6-well plate at 1×10⁶ cells/ml; 2ml of cell suspension was added to each well; and the cortical neurons were cultivated to the 7th day. The grouping and treatment methods were as described above. After reoxygenation and resugaring for 24h, the cell culture medium was discarded, placed on ice, and rinsed twice with PBS (IX). 80µl of cell protein lysate (mammalian protease inhibitor: mammalian cell protein extraction reagent, 1:100) was added to each well, and lysed on ice for 30 min. The substances in the wells were scraped with a protein scraper and transferred to a 1.5ml centrifuge tube. The centrifuge tube was shaken for about 1 min on a vortex oscillator. 1.5ml centrifuge tube was placed in a 4°C centrifuge and centrifuged at 12,000 rpm for 30min; and the supernatant was transferred into a new 0.6ml centrifuge tube to obtain the total cell protein.

Extraction of mitochondrial protein and removal of mitochondrial cytoplasmic protein: the cortical neurons were inoculated in the 6-well plate at 1×10⁶ cells/ml; and 2ml of cell suspension was added to each well. The cortical neurons were cultured to the 7th day. The grouping and treatment methods were as described above. The culture medium was discarded after 24h. The cells were rinsed with PBS (IX) and then added with 0.25% trypsin to digest the adherent cortical neurons. When the neurites were contracted, the trypsin was discarded and the DMEM complete medium was added to terminate the digestion. The neurites were centrifuged at room temperature and 100 g for 5-10 min; the supernatant was discarded; ice-bath PBS (IX) was added to resuspend the rinsed cells; 10µl of cell suspension was used to count with a hemocytometer; the remaining cells were centrifuged at 4°C and 600g for 5min; the cells were gently resuspended based on that every 2-5×10⁷ cells were added with 1-2.5ml of mitochondrial separation reagent containing 1mM phenylmethylsulfonyl fluoride (PMSF), and placed on ice for 10-15min; and the cell suspension was transferred into an appropriate glass homogenizer. After homogenizing on ice for a certain number of times, an appropriate amount of the homogenate was stained with trypan blue, and observed under a microscope; and homogenizing was stopped until the proportion of blue cells, i.e., trypan blue stained positive cells, exceeded 50%. The homogenate was centrifuged at 4°C and 1000g for 10 min to settle unlysed cells, membrane debris and cell nuclei; the supernatant was transferred and centrifuged at 4°C and 3500g for 10 min to settle the mitochondria; the supernatant was transferred into another centrifuge tube, and centrifuged at 4°C and 12,000g for 10 min to separate the supernatant to remove mitochondrial cytoplasmic protein. The precipitate was mitochondria, and added with 150-200µl of mitochondrial lysate containing 1 mM of PMSF for lysis to obtain mitochondrial protein.

Extraction of nuclei and cytoplasmic protein: the cortical neurons were inoculated into the 6-well plate at 1×10⁶ cells/ml, and 2ml of cell suspension was added to each well. The cortical neurons were cultivated to the 7th day. The grouping and treatment methods were as described above. The culture medium was discarded after 24h. The cortical neurons were rinsed once with PBS (IX); the cells were scraped with the cell scraper and centrifuged; the cells were collected, and vortexed for a few seconds based on that 200µl of cytoplasmic protein extraction reagent A containing 1 mM of PMSF was added into every 20µl of cells until precipitated cells were completely dispersed and placed in ice for 10-15 min; cytoplasmic protein extraction reagent B was added to vortex for 5 s; then the cells were centrifuged at 4°C and 12,000-16,000g for 5min; and the supernatant was transferred into another centrifugal tube to obtain the cytoplasmic protein. 50µl of nucleoprotein extraction reagent containing 1 mM of PMSF was added to the precipitate; the precipitate was vortexed until the precipitate was dispersed and placed in ice for 30 min; in the process, the precipitate was taken out every 1-2min and vortexed for 15-30s, and centrifuged at 4°C and 12,000-16,000g for 10min. The supernatant was transferred into another centrifuge tube to obtain the nuclear protein.

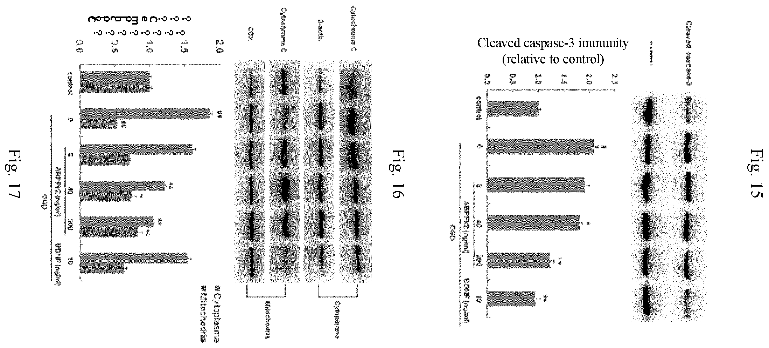

Western blot analysis: the protein concentration was measured by BCA method; SDS-PAGE protein loading buffer (5X) was added into the protein sample to dilute to IX; the solution was shaken and boiled for 5 min in a metal bath. 10µl of supernatant was respectively taken for 12% SDS-PAGE. The supernatant was wet-transferred to a PVDF membrane in an ice water bath. After trarsmembrane, the supernatant was rinsed with 25ml of TBS/T for 5 min at room temperature, and the PVDF membrane was put into TBS/T containing 5% skimmed milk powder and sealed at room temperature for 1 h. Primary antibodies were diluted with 5% skimmed milk powder. Except for GADPH 1:10000, Bax 1:2000 and Cyt C 1:500 dilution, HI, COX, AIF, Bcl-2, Cleaved Caspase-3, Akt and p-Akt were diluted at 1:1000 and incubated overnight at 4°C on a shaker. TBS/T was used for rinsing for 5 min×3 times. Secondary antibodies were diluted with TBS-T, Anti-Mouse IgG H&L (HRP) 1:10000 and Anti-Mouse IgG H&L (HRP) 1:20000, and incubated in the shaker at room temperature for 2h. TBS/T was used for washing for 5 min×5 times. The PVDF membrane was put into an automatic chemiluminescence/fluorescence image analysis system, and BeyoECL Star was added on the membrane to conduct gray membrane scanning. Image J was used for gray statistics; Cyt C used COX or β-actin as internal control, AIF used H1 or COX as internal control, p-AKT used AKT as internal control, and other proteins used GAPDH as internal control for comparative analysis. The experiment was repeated for 3 batches of protein samples, and each batch of protein samples was repeated for 3 times. The results showed that compared with the normal control group, the expression of anti-apoptotic protein Bcl-2 in the total protein of cortical neuron cells after OGD damage was significantly down-regulated, and the expressions of pro-apoptotic proteins Bax and Cleaved caspase-3 were significantly up-regulated (#P<0.05 and ##P<0.01). Compared with the OGD damage group, the Bcl-2 expression of the ABPPk2 high-dose group was significantly up-regulated, while the expressions of pro-apoptotic proteins Bax and Cleaved caspase-3 in the medium-dose group and the high-dose group were significantly down-regulated (^{∗}P<0.05 and ^{∗∗}P<0.01), as shown in Fig. 15 and Fig. 16.

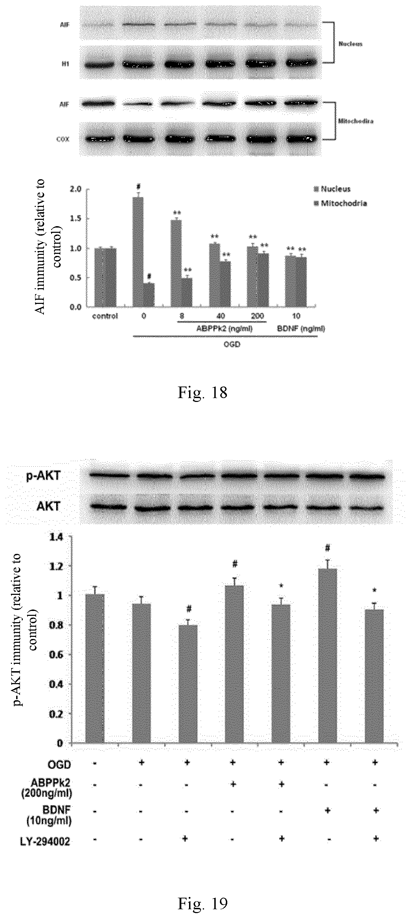

Compared with the normal control group, the expressions of Cyt C and AIF in the mitochondria of cortical neurons after OGD damage were significantly down-regulated, and the expressions of Cyt C in the cytoplasma and AIF in the nucleus were significantly up-regulated (#P<0.05 and ##P<0.01). After treatment with ABPPk2, the expression of Cyt C in mitochondria was significantly up-regulated, the expression of Cyt C in cytoplasma was significantly down-regulated (as shown in Fig. 17), the expression of AIF in mitochondria was significantly up-regulated, and the expression of AIF in the nucleus was significantly down-regulated (^{∗} P<0.05 and ^{∗∗}P<0.01) (as shown in Fig. 18). It is suggested that ABPPk2 can down-regulate the expression of pro-apoptotic proteins in the cortical neurons after OGD damage, up-regulate the expression of anti-apoptotic protein, inhibit the release of Cyt C of the mitochondria of cortical neurons after OGD damage into the cytoplasma, inhibit the release of AIF of the mitochondria of the cortical neurons after OGD damage into the nucleus, and then inhibit the apoptosis of the cortical neurons induced by OGD damage.

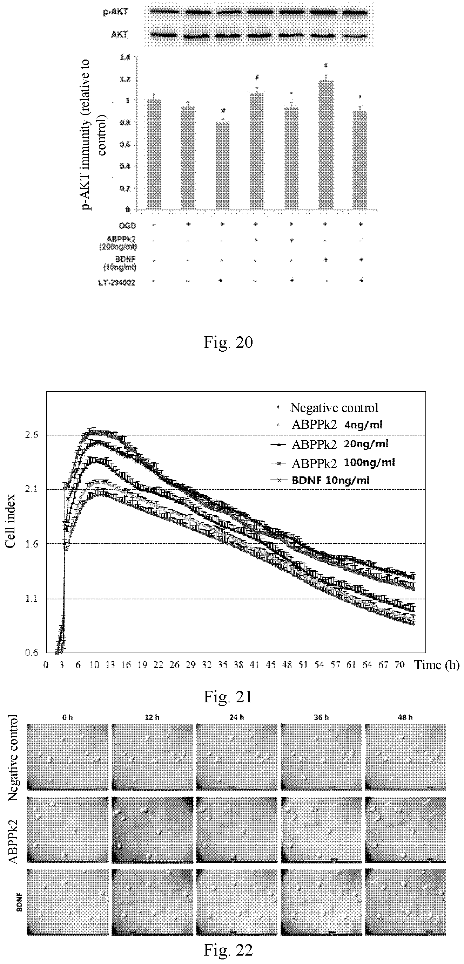

The PI3K inhibitor LY-294002 was added to the cortical neuron culture system after OGD damage for treatment for 30 min, and then different concentrations of ABPPk2 and BDNF were added for treatment. Reoxygenation and resugaring were conducted for 24 h. Western Blot was used to detect and analyze the expressions of p-AKT and Cleaved caspase-3. The results showed that compared with the normal control group, the expression of p-AKT was down-regulated after OGD damage, and the expression of p-AKT was significantly up-regulated after treatment with ABPPk2 at high dose(P<0.05). After OGD and LY-294002 treatment, compared with the OGD group, the expression of p-AKT was significantly down-regulated (P<0.05), while the expression of p-AKT in the ABPPk2 high-dose group was significantly up-regulated (P<0.05) (as shown in Fig. 19). Compared with the normal control group, the expression of Cleaved caspase-3 was significantly up-regulated after OGD damage (P<0.05), and the expression of Cleaved caspase-3 was significantly down-regulated after treatment with ABPPk2 at high dose (P<0.01). After OGD and LY-294002 treatment, compared with the OGD group, the expression of Cleaved caspase-3 was significantly up-regulated (P<0.01), while the expression of Cleaved caspase-3 in the ABPPk2 high-dose group was significantly down-regulated (P<0.01) (Fig. 20). It is suggested that ABPPk2 may antagonize the apoptosis of the cortical neurons induced by OGD damage through the PI3K/AKT signaling pathway, and then have a protective effect on the cortical neurons induced by OGD damage.

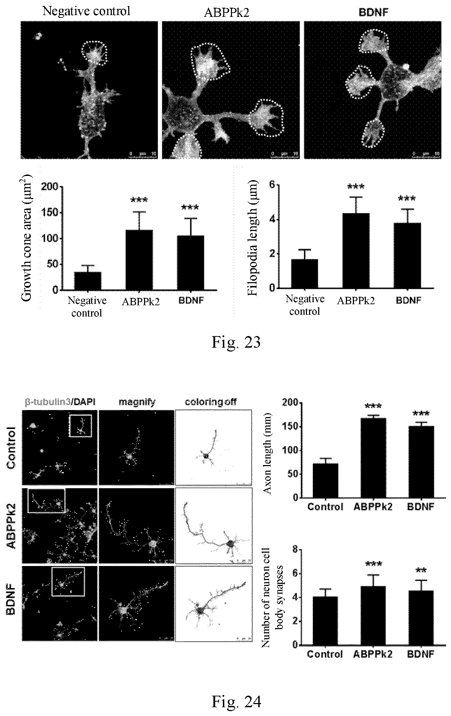

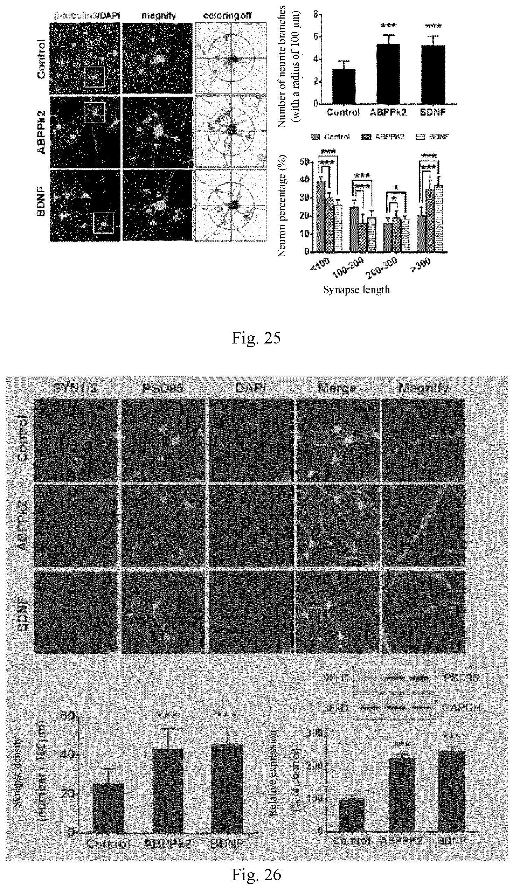

Embodiment 8: Effect of ABPPk2 on growth of primary cultured hippocampal neurons
(1) Primary culture of fetal rat hippocampal neurons: after a rat pregnant for 17 days was anesthetized, a fetal rat was taken out under sterile conditions; the brain of the fetal rat was taken out on ice; the hippocampus was carefully taken out under a dissecting microscope; and the blood vessel membranes were removed. 0.125% trypsin/EDTA was digested at 37°C for 10 min, and the digestion was stopped by adding the DMEM complete medium containing 10% FBS. Cell debris were removed through centrifugal at 1000 rpm for 5 min; the DMEM complete medium was added to resuspend the cells; the cells were gently blown until a single cell suspension was formed; and the cells were inoculated at an appropriate density on a culture plate (dish) pre-coated with polylysine (PLL) and cultured at 37°C, 5% CO₂ incubator. After 4 h, the cells were adhered to the wall; the DMEM complete medium was discarded and replaced with neurobasal medium containing different concentrations of ABPPk2 (the concentrations of ABPPk2 were 4 ng/ml, 20 ng/ml and 100 ng/ml); the neurobasal medium containing 10 ng/ml BDNF was used as the positive control group, and neurobasal was used as the negative control group.
(2) Neuron growth was observed by a label-free real time cell analysis (RTCA) system: the hippocampal neuron single cell suspension obtained in (1) was inoculated on an E-plate pre-coated with PLL at a cell density of 1×10⁶. After 4 h, the cells were adhered to the wall; the DMEM complete medium was discarded and replaced with the neurobasal medium containing different concentrations of ABPPk2 (the concentrations of ABPPk2 were 4 ng/ml, 20 ng/ml and 100 ng/ml); the neurobasal medium containing 10 ng/ml BDNF was used as the positive control group, and neurobasal was used as the negative control group to observe the cell growth curve in real time. The higher the cell index is, the better the cell growth is, and the more neuron cell body growth and axonal extension are for the neurons. Each experimental group included 2 complex holes, and the experiment was repeated for 3 times. The results showed that treatment with ABPPk2 can promote the growth of the hippocampal neurons and the extension of neurites, as shown in Fig. 21.
(3) Neuron growth was observed by a live cell station: the hippocampal neuron single cell suspension obtained in (1) was inoculated on a culture plate pre-coated with PLL at a cell density of 5×10⁵. After 4 h, the cells were adhered to the wall; the DMEM complete medium was discarded and replaced with the neurobasal medium containing 100 ng/ml ABPPk2; the neurobasal medium containing 10 ng/ml BDNF was used as the positive control group, and neurobasal was used as the negative control group; the culture plate was placed in the cell culture chamber of the live cell station and continuously observed for 48 h; and the neurons under the field of view were automatically photographed every two minutes. Each experimental group included 2 complex holes, and the experiment was repeated for 3 times. The results showed that treatment with ABPPk2 can promote the growth of the hippocampal neurons and the extension of neurites, as shown in Fig. 22.
(4) The immunocytochemical staining method was used to observe the effect of ABPPk2 on the growth cones of primary fetal rat hippocampal neuron neurites. Primary hippocampal neurons were cultured according to the steps in (1), and the cells were inoculated on small round slides pre-coated with PLL at a density of 5×10⁴, and then placed on a 24-well culture plate for culturing. Grouping and treatment were as described above. After 24 h, double-labeled immunocytofluorescent chemical staining of β-tubulin3 (TRITC) and F-actin (FITC) was conducted; the growth of the neurons was observed under a laser confocal microscope; and the area of the neuron growth cone and the length of filopodia were counted, as shown in Fig. 23. The results showed that after the effect of ABPPk2 for 12 h, the area of the growth cone can be significantly increased and the length of the filopodia can be increased (compared with the negative control group, ^{∗∗∗}P<0.001).
(5) The immunocytochemical staining method was used to observe the effect of ABPPk2 on the growth of primary fetal rat hippocampal neuron neurites. Primary hippocampal neurons were cultured according to the steps in (1), and the cells were inoculated on small round slides pre-coated with PLL at a density of 5×10⁴, and then placed on a 24-well culture plate for culturing. Grouping and treatment were as described above. After 24 h, β-tubulin3 immunocytochemical staining was conducted; the growth of the neurites was observed under the laser confocal microscope; and the length of the neurites and the number of the neurites from the neuron cell bodies were counted. The length of the neurites started from the axon hillock of the cell body to the top of the axon. The number of the neurites from the neuron cell bodies refers to the neurites directly connected with the cell bodies, as shown in Fig. 24. The results showed that after the effect of ABPPk2 for 24 h, the extension of hippocampal neuronal neurites can be significantly promoted and the number of the neurites from the neuron cell bodies can be significantly increased (compared with the negative control group, ^{∗∗∗}P<0.001).
(6) β-tubulin3 immunocytochemical staining was conducted on the hippocampal neurons after 48 h of drug action; the growth of the neurites was observed under the laser confocal microscope; the number of neurite branches and the proportion of the neurites with different lengths were counted; and the number of neurite branches refers to the number of branches of the neurites in a circle with a neuron cell body as the center and a radius of 100 µm. The results were shown in Fig. 25. After the effect for 48 h, the neurite branches of the hippocampal neurons in the ABPPk2 treatment group were increased significantly, and the proportion of long neurite neurons was significantly higher than that of the negative control group (compared with the negative control group, ^{∗∗∗}P<0.001 and ^{∗}P<0.05).
(7) Promotion effect of ABPPk2 on synapse formation in primary cultured hippocampal neurons. The primary hippocampal neurons were cultured as described above. The cells were treated with drugs for 8 d; double-labeled immunofluorescence cytochemical staining of the presynaptic membrane marker protein Synaptotagamin1/2 (SYN) and postsynaptic membrane marker protein post synapse density protein 95 (PSD95) was performed; and a cristula co-localized by SYN and PSD95 was a synaptic structure. The field of view was randomly selected and photographed under the laser confocal microscope, and the number of synaptic cristulas on every 100 µm neurites was counted. The effect of ABPPk2 on the expression of PSD95 was analyzed by Western blot. The steps were as follows: the total protein was extracted and the total protein concentration was determined by Bradford method. The protein supernatant was dissolved in 1×SDS loading buffer, boiled for 5 min, and subjected to 12% SDS-PAGE electrophoresis. The protein was wet-transferred to the PVDF membrane, closed with 5% skim milk powder, and then added with the primary antibody (mouse anti monoclonal PSD95, 1:1000) and the secondary antibody (HRP goat-anti-mouse IgG, 1:200) in sequence. GAPDH (1:200) was used as the internal control, and finally the ECL method was used for color developing, tabletting, exposure and development. GS800 Calibrated Densitometer scanner (BIO-RAD) was used for gray scanning, and PDQuest7.2.0 software was used for analysis. The results were shown in Fig. 26. Through treatment with ABPPk2 for 8 d, the number of the formed synapses can be significantly increased and the expression of PSD95 protein was up-regulated.

Embodiment 9: Three-dimensional spatial structure of multi-disulfide-bond long-chain peptide in homology modeling mode
For the sequence CLETSGSCIPGAQHNCCSGVCVPLVTLFYGVCY of the multi-disulfide-bond long-chain peptide, homology search (BLAST) was conducted in the PDB. The results showed that A chain fragment of the protein crystal structure with PDB number of 1Q3J had the highest homology with this sequence and the overall structural similarity was 48% (see Scheme1), and the coverage rate was also ideal (96%). Therefore, the crystal structure (1Q3J_A) was selected as a template for homology modeling of the sequence structure.

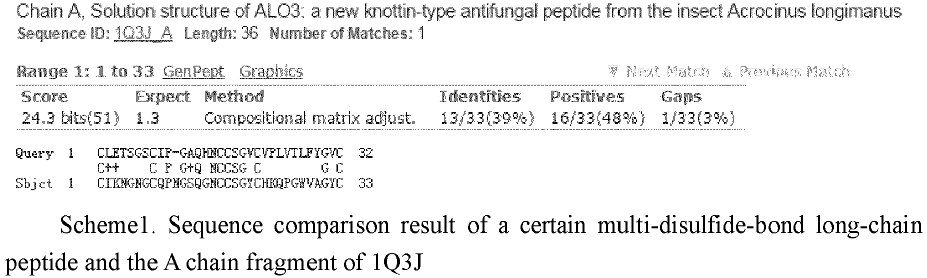

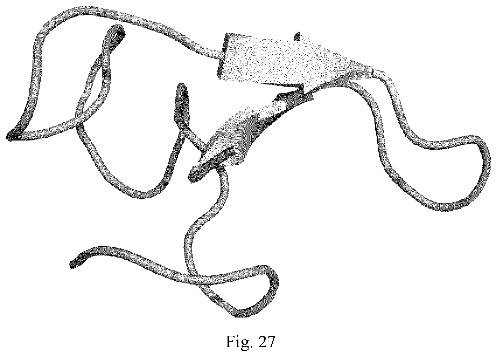

The SWISS-MODEL tool was used to construct the homology modeling of the 3D structure of the polypeptide of this sequence. Due to the limitation of the template structure, the sequence structure obtained by homology modeling only had 1-32 residues, and Tyr33 was missing. Here, Tyr33 was firstly supplemented; and then molecular dynamics simulation software Amber was used to optimize the sequence structure. The simulation time was 20ns. After water molecules and Na ions were deleted, the corresponding PDB file was saved. The PDB file was opened by Pymol software. Fig. 27 showed the three-dimensional structural model of the sequence after molecular dynamics optimization. It can be seen from the figure that the three-dimensional structure of the sequence mainly included the loop structure, and also included two shorter β-sheet structures (composed of Cys21-Pro23 and Gly30-Cys32 respectively).

Embodiment 10: Synthesis and activity research of ABPPk2 derivative
The above multi-disulfide-bond long-chain peptide with the changed sequence was synthesized by shanghai Bootech Company. The synthesis process used the Fmoc/tBu strategy, and the peptide was synthesized in a solid phase on Fmoc-AM-MBHA resin (Barany,G and Merrifield,R.B.J Am Chem Soc.99(1977)7363-7365). The amino acid was coupled using activation method of DIC/HOBt, and the completion of the coupling reaction was verified by a ninhydrin test (Kaiser, E.,Colescott,R.L., Bossinger,C.D.,Cook, P.I. Anal Biochem.34(1970)595-598). The peptide was separated from the resin by treatment with TFA/EDT/H₂O/TIS (94%/2.5%/2.5%/1%) solution, precipitated with ether, and freeze-dried for 72 hours. A disulfide bridge was formed by oxidation with dimethyl sulfoxide (DMSO) to achieve cyclization (Peptide Synthesis Protocols, Methods in Molecular Biology, Totowa, NJ, 1994, pages 91-169), and purified by RP-HPLC. The collected fractions were independently analyzed on analytical RP-HPLC, and the fractions with purity greater than 95% were combined to manufacture the final preparation of ABPPk2 derivative. The multi-disulfide-bond long-chain peptide with the sequence CLETSGSCIPGAQHNCCSGVCVPLVTLFYGVCY was obtained, and the connection modes of the disulfide bonds were CysI-CysIV, CysII-CysV and CysIII-CysVI. The multi-disulfide-bond long-chain peptide was used to experiment on the effect of primary hippocampal neuron NMDA damage, and the experimental solution was the same as embodiment 6. Experimental results showed that the multi-disulfide-bond long-chain peptide can effectively reduce the decrease of hippocampal neuron activity caused by NMDA damage, thereby proving that the modified polypeptide has a protective effect on primary hippocampal neuron damage caused by NMDA.

The technical features of the above embodiments can be arbitrarily combined. To make the description concise, all possible combinations of the technical features in the above embodiments are not described. However, as long as the combinations of the technical features do not have contradictions, the combinations shall be considered to be the scope disclosed in this description.

The above embodiments only express several implementation modes of the present invention, and are described more specifically in details, but shall not be consequently interpreted as a limitation to the scope of the patent for invention. It should be noted that, for those ordinary skilled in the art, several deformations and improvements can also be made without departing from the concept of the present invention, all of which belong to the protection scope of the present invention. Therefore, the protection scope of the patent of the present invention shall be subject to appended claims.

SEQUENCE LISTING

<110>  DALIAN INSTITUTE OF CHEMICAL PHYSICS, CHINESE ACADEMY OF
       SCIENCES
       NANTONG UNIVERSITY

<120>  MULTI-DISULFIDE-BOND LONG-CHAIN PEPTIDE WITH NEUROPROTECTIVE
ACTIVITY, PHARMACEUTICAL COMPOSITION AND APPLICATION

<140>  2018114116980
<141>  2018-11-25

<160>  2

<170>  SIPOSequenceListing 1.0

<210>  1
<211>  0
<212>  PRT
<213>
Cys-Leu-Glu-Ser-Gly-Thr-Ser-Cys-Ile-Pro-Gly-Ala-Gln-His-Asn-Cys-Cys-Ser-Gly-V
al-Cys-Val-Pro-Ile-Val-Thr-Ile-Phe-Tyr-Gly-Val-Cys-Tyr

<400>  1

<210>  2
<211>  0
<212>  PRT
<213>
Cys-X1-Ser-Cys-X2-Gln-His-Asn-Cys-Cys-Ser-Gly-Val-Cys-Val-X3-Phe-Tyr-Gly-Val-
Cys-Tyr

<400>  2

## Claims

1. A multi-disulfide-bond long-chain peptide with neuroprotective activity, **characterized in that** the amino acid sequence of polypeptide is Cys-X1-Ser-Cys-X2-Gln-His-Asn-Cys-Cys-Ser-Gly-Val-Cys-Val-X3-Phe-Tyr-Gly-Va l-Cys-Tyr wherein
X1 is a tetrapeptide, pentapeptide or hexapeptide sequence connected by 4-6 amino acids successively, and the amino acids are selected from 1-6 of Ser, Gly, Leu, Lys and Thr;
X2 is a tetrapeptide, pentapeptide or hexapeptide sequence connected by 4-6 amino acids successively, and the amino acids are selected from 1-5 of Gly, Ala, Ile, Arg and Pro;
X3 is a tetrapeptide, pentapeptide or hexapeptide sequence connected by 4-6 amino acids successively, and the amino acids are selected from 1-5 of Thr, Leu, Ile, Pro and Val.

2. The multi-disulfide-bond long-chain peptide according to claim 1, **characterized in that** the amino acid sequence of the polypeptide is the amino acid sequence of ABPPk2, Cys-Leu-Glu-Ser-Gly-Thr-Ser-Cys-Ile-Pro-Gly-Ala-Gln-His-Asn-Cys-Cys-Ser-Gly-V al-Cys-Val-Pro-Ile-Val-Thr-Ile-Phe-Tyr-Gly-Val-Cys-Tyr
(i.e., CLESGTSCIPGAQHNCCSGVCVPIVTIFYGVCY).

3. The multi-disulfide-bond long-chain peptide according to claim 1 or 2, **characterized in that** three pairs of disulfide bonds are connected in CysI-CysIV (that is, the sulfur in the first Cys is connected with the sulfur in the fourth Cys to form a disulfide bond), CysII-CysV (that is, the sulfur in the second Cys is connected with the sulfur in the fifth Cys to form a disulfide bond), and CysIII-CysVI (that is, the sulfur in the third Cys is connected with the sulfur in the sixth Cys to form a disulfide bond).

4. The multi-disulfide-bond long-chain peptide according to claim 1, 2 or 3, **characterized in that** a three-dimensional spatial structure comprises two or three pairs of β-antiparallel structures which are located at N-terminal, a middle sequence and C-terminal of the multi-disulfide-bond long-chain peptide.

5. The multi-disulfide-bond long-chain peptide according to claim 2, **characterized in that** interaction sites between the ABPPk2 and NMDA2B receptor are reserved, i.e., Gln13, Asn15, Phe28 and Tyr33.

6. The multi-disulfide-bond long-chain peptide according to claim 1, **characterized in that** the peptide comprises a linear peptide formed by an amino group and a carbonyl group at the N-terminal and the C-terminal of the peptide, or a cyclic peptide formed by peptide bonds between the amino group and the carbonyl group at the N-terminal and the C-terminal of the peptide.

7. The multi-disulfide-bond long-chain peptide according to claim 1, **characterized in that** a disulfide covalent bond comprises groups X1, X2 and X3 and cysteines in the fixed sequence in addition to the groups; and when two cysteines are separated by more than nine amino acid residues, the corresponding cysteines form the disulfide covalent bond.

8. The multi-disulfide-bond long-chain peptide according to claim 1 or 7, **characterized in that** a glycosidic bond comprises groups X1, X2 and X3, and side chain amino groups (Lys and Arg), carbonyl groups (Glu and Asp) and amide groups (Gln and Asn) in the fixed sequence in addition to the groups; the amino groups, the carbonyl groups or the amide groups can form glycosidic bonds with the following monosaccharides or disaccharides; the monosaccharides comprise one or more than one of arabinose, ribose, xylose, lyxose, glucose, mannose, fructose and galactose; and the disaccharides comprise one or more than one of maltose, lactose, sucrose, trehalose, cellobiose, gentiobiose and melibiose.

9. The multi-disulfide-bond long-chain peptide according to claim 1, **characterized in that** the N-terminal of the polypeptide is covalently linked to a pyroglutamic acid or acetyl group, and the acetyl group comprises one or more than one of acetylcholine, paracetamol, acetylsalicylic acid, acetic acid, acetamide and acetic anhydride.

10. A pharmaceutical composition, comprising at least one multi-disulfide-bond long-chain peptide of any one of claims 1-9.

11. The pharmaceutical composition according to claim 10, **characterized in that** the pharmaceutical composition further comprises a pharmaceutically acceptable solvent, excipient or carrier.

12. An application of the multi-disulfide-bond long-chain peptide of any one of claims 1-9 in preparation of a central nerve protective agent for treating ischemic stroke.

13. An application of the multi-disulfide-bond long-chain peptide of any one of claims 1-9 in preparation of a drug for treating neuronal hypoxic-ischemic damage.

14. An application of the multi-disulfide-bond long-chain peptide of any one of claims 1-9 in preparation of a drug for promoting growth of hippocampal neurons.

15. The application according to any one of claims 12-14, **characterized in that** the drug further comprises a pharmaceutically acceptable solvent, excipient or carrier.
